# EUROPEAN PATENT SPECIFICATION

(11) **EP 3 996 583 B1**
(45) Date of publication and mention of the grant of the patent: **25.02.2026**
(21) Application number: 20837310.0
(22) Date of filing: 08.07.2020
(51) Int. Cl.: G01N 33/483, C12Q 1/18

(54) **METHOD AND SYSTEM FOR DETERMINING ANTIMICROBIAL RESISTANCE OF A SAMPLE USING SURFACE TOPOGRAPHY DETERMINED FROM OPTICAL IMAGING DATA**
VERFAHREN UND SYSTEM ZUR BESTIMMUNG DER ANTIMIKROBIELLEN RESISTENZ EINER PROBE UNTER VERWENDUNG DER AUS OPTISCHEN BILDGEBUNGSDATEN ERMITTELTEN OBERFLÄCHENTOPOGRAPHIE
PROCÉDÉ ET SYSTÈME PERMETTANT DE DÉTERMINER LA RÉSISTANCE ANTIMICROBIENNE D'UN ÉCHANTILLON À L'AIDE DE LA TOPOGRAPHIE DE SURFACE DÉTERMINÉE À PARTIR DE DONNÉES D'IMAGERIE OPTIQUE

(30) Priority: 08.07.2019 US 201962871687 P
(43) Date of publication of application: 18.05.2022
(73) Proprietor: Emory University, Atlanta, Georgia 30322 (US); Georgia Tech Research Corporation, Atlanta, GA 30318 (US)
(72) Inventor: WEISS, David, Atlanta, Georgia 30322 (US); HUFNAGEL, David, Atlanta, Georgia 30322 (US); YUNKER, Peter, Atlanta, Georgia 30332 (US); KALZIQI, Arben, Atlanta, Georgia 30332 (US)
(74) Representative: Harden, Henry Simon
(86) International application number: PCT/US2020/041182
(87) International publication number: WO 2021/007312

(56) References cited:
- WO-A1-01/09371
- WO-A1-2019/063690
- US-A1- 2011 104 725
- US-A1- 2015 337 352
- US-A1- 2016 102 334
- US-A1- 2016 215 324
- US-A1- 2017 298 408
- US-A1- 2018 010 084

## Description

### CROSS-REFERENCE TO RELATED APPLICATIONS

This application claims the benefit of U.S. Provisional Application No. 62/871,687 filed July 8, 2019.

### BACKGROUND

Antimicrobial, such as antibiotic, resistance is a significant threat to public health. Currently, most diagnostics to determine one antimicrobial susceptibility, antibiotics, require long incubation times. While the clinician waits for the antibiotic susceptibility results, the clinician can be forced to treat patients with a broad spectrum and aggressive antibiotics, resulting in inappropriate therapy and poor patient outcomes. This overprescription of antimicrobials, in particular antibiotics, generally has also contributed to the emerging problem of drug-resistant bacteria.

WO 2019/063690 A1 discloses an imaging system and method which provides automated microbial growth detection for antibiotic sensitivity testing. A processing system having an image sensor for capturing images of an inoculated culture plate having antibiotic disks disposed on the culture media captures images of the plate at separate times (e.g., first and second images).

### SUMMARY

Thus, there is need for rapid and sensitive diagnostics to accurately determine antimicrobial susceptibility.

The disclosure relates to a method and a system in accordance with the independent claims land 15 respectively that can quickly and automatically determine susceptibility of a sample to one or more antimicrobial agents using one or more metrics determined from the surface topography of a region of the sample. This could result in the right medication regimen being administered to the patient in a timely manner, thereby improving patient care, patient outcomes, as well as could help address the drug-resistant bacteria problem.

In accordance with the present invention the method of claim 1 and the system of claim 15 includes determining a level of susceptibility of a sample to one or more antimicrobial agents based on topographic surface properties. The method of claim 1 includes providing a container including one or more sites. The one or more sites include a sample having one or more microorganisms and one or more concentrations of one or more antimicrobial agents. The method and system includes receiving a topographic surface profile for one or more regions of each site The one or more regions include at least one region having the sample and a concentration of the one or more antimicrobial agents. The method and system includes determining one or more metrics of at least one region of the one or more regions of each site using the topographic surface profile for each site. The one or more metrics include one or more of volumetric metrics, distribution metrics, spatial correlation metrics, among others, or a combination thereof. The method and system includes determining one or more indices representing a level of susceptibility of the sample to the concentration of the one or more antimicrobial agents provided in each site using the one or more metrics for the at least one region of each site from one or more indices.

In some embodiments, the one or more indices may include one or more qualitative indices, the one or more qualitative indices including a first index indicating that the sample is susceptible to the concentration of the one or more antimicrobial agents and a second index indicating that the sample is resistant to the concentration of the one or more antimicrobial agents. In some embodiments, the one or more qualitative indices may include a third index indicating that the sample is heteroresistant to the concentration of the one or more antimicrobial agents.

In some embodiments, the one or more antimicrobial agents may include one or more antibiotic agents.

In some embodiments, each sit may include a culture medium. The culture medium may be a solid and/or liquid medium. In some embodiments, the culture medium may be a solid culture medium. In some embodiments, the culture medium may include an agar pad and/or a polyacrylamide gel.

In some embodiments, the determining the one or more metrics may use pixels of the associated region of a topographic image acquired by an optical imaging device. In accordance with the invention, the topographic surface profile includes a height value for each pixel disposed within the associated region. In accordance with the invention, the one or more metrics is determined using the height value for each pixel disposed within the associated region.

In some embodiments, the one or more volumetric metrics may include a total volume of at least one region. In some embodiments, the total volume may be for the entire site. In some embodiments, the one or more distribution metrics may include kurtosis of at least one region and/or entire site.

In some embodiments, the one or more qualitative may be determined based on a biophysical relationship between the one or more metrics and the one or more indices.

In some embodiments, the one or more sites may include at least one site and/or at least one section that includes the sample without the one or more antimicrobial agents. In some embodiments, the method may include determining one or more metrics of the at least one site and/or the at least one section that includes the sample without the one or more antimicrobial agents, the one or more metrics including one or more of volumetric metrics, distribution metrics, spatial correlation metrics, among others, or a combination thereof. In some embodiments, the determining the one or more qualitative indices may include comparing the one or more metrics of the one or more sites including the sample and the one or more antimicrobial agents to the one or more metrics of the at least one site and/or the at least one section that includes the sample without the one or more antimicrobial agents.

In some embodiments, the one or more metrics may include determining one or more volume metrics.

In some embodiments, each site may include one or more test sections and/or one or more control sections. Each test section may have the sample and a concentration of one or more antimicrobial agents disposed on a culture medium. Each control section may have the culture medium without the one or more antimicrobial agents. In some embodiments, each control section may be bare (i.e., without the sample).

In some embodiments, the method may further include obtaining raw topographic data of one or more regions of the test section and/or the control section using optical imaging. The method may further include calibrating the raw topographic data for the one or more regions of the test section with the raw topographic image data for the one or more regions of the control section. In some embodiments, the method may include generating the topographic profile for one or more regions of the test section of each site.

In some embodiments, the topographic profile may be represented by a topographic map.

In some embodiments, the container may include a first site having the sample and a first concentration of one or more antimicrobial agents and a second site having a different concentration of the one or more antimicrobial agents and/or one or more different antimicrobial agents. The determining the one or more indices may include determining one or more indices for the first site and determining one or more indices for the second site.

In some embodiments, the one or more indices may include one or more of qualitative and/or quantitative indices for each site. In some embodiments, a measure of confidence may be determined for each qualitative index of the one or more qualitative indices. In some embodiments, the one or more quantitative indices for each site may be determined using the one or more qualitative indices determined for each site. In some embodiments, another one or more qualitative indices may be determined using the one or more quantitative indices for each site and/or the measure of confidence corresponding to the one or more qualitative indices. In some embodiments, the one or more quantitative indices may include a quantitative value corresponding to a fraction of resistant cells disposed in at least a region of the test section of each site.

In accordance with the invention the system determines a level of susceptibility of a sample disposed in one or more sites of a container to one or more antimicrobial agents based on topographic surface properties. The system includes at least one processor; and a memory. In some embodiments, the one or more sites may include a sample having one or more microorganisms and one or more concentrations of one or more antimicrobial agents. The processor is configured to receive a topographic surface profile for one or more regions of each site. The one or more regions may include at least one region having the sample and a concentration of the one or more antimicrobial agents. The processor is further configured to determine one or more metrics of at least one region of the one or more regions of each site using the topographic surface profile for each site. The one or more metrics may include one or more of volumetric metrics, distribution metrics, spatial correlation metrics, among others, or a combination thereof. The processor is configured to determine one or more indices representing a level of susceptibility of the sample to the concentration of the one or more antimicrobial agents provided in each site using the one or more metrics for the at least one region of each site from one or more indices.

Additional advantages of the disclosure will be set forth in part in the description which follows, and in part will be obvious from the description, or may be learned by practice of the disclosure. The advantages of the disclosure will be realized and attained by means of the elements and combinations particularly pointed out in the appended claims. It is to be understood that both the foregoing general description and the following detailed description are exemplary and explanatory only and are not restrictive of the disclosure, as claimed.

### BRIEF DESCRIPTION OF THE DRAWINGS

The disclosure can be better understood with the reference to the following drawings and description. The components in the figures are not necessarily to scale, emphasis being placed upon illustrating the principles of the disclosure.
Figure 1 shows an example of a system for determining a susceptibility of a sample to one or more antimicrobial agents using topographic data;
Figure 2 shows a method of determining one or more indices indicating a level of susceptibility of a sample to one or more antimicrobial agents using topographic data according to embodiments;
Figure 3 shows an example of a sample site according to embodiments;
Figure 4 shows an example of a numerical integration according to embodiments.
Figure 5 shows an example of a relationship of a qualitative susceptibility index using curvature according to embodiments;
Figure 6 shows an example of a relationship of a qualitative susceptibility index using kurtosis according to embodiments;
Figure 7 shows an example of a method of determining a qualitative susceptibility index using topographic data according to embodiments;
Figure 8A shows examples of topographic maps from Carbapenem-resistant *Klebsiella* isolates with different qualitative susceptibility indices to colistin determined according to embodiments, and Figure 8B shows a relationship between the qualitative index and metric (e.g., height) determined according to embodiments;
Figure 9 shows an example of a method of determining a quantitative susceptibility index using topographic data according to embodiments;
Figure 10A shows an example of a height histogram determined for Carbapenem-resistant Klebsiella isolates, and Figure 10B shows an example of a quantitative index and the associated quantitative-based qualitative index determined using the height histogram determined in Figure 10A according to embodiments; and
Figure 11 shows a block diagram illustrating an example of a computing system according to embodiments.

### DESCRIPTION OF THE EMBODIMENTS

In the following description, numerous specific details are set forth such as examples of specific components, devices, methods, etc., in order to provide a thorough understanding of embodiments of the disclosure. It will be apparent, however, to one skilled in the art that these specific details need not be employed to practice embodiments of the disclosure. In other instances, well-known materials or methods have not been described in detail in order to avoid unnecessarily obscuring embodiments of the disclosure. While the disclosure is susceptible to various modifications and alternative forms, specific embodiments thereof are shown by way of example in the drawings and will herein be described in detail.

The system and method of the disclosure can rapidly determine a level of susceptibility of one or more microorganisms provided in a sample to one or more antimicrobial agents, such as one or more antibiotics, and thereby better guiding treatment of a patient. For example, the systems and methods use optical imaging, such as interferometry, to rapidly determine one or more antimicrobials agents to which a given sample could be resistant or susceptible.

In some examples, the method and system can also determine one or more antimicrobials agents to which given a given sample could be antimicrobial(s) heteroresistant, which has not been provided in readily available diagnostics. Heteroresistance can lead to clinicians inappropriately and unknowingly treating patients with antibiotics or antimicrobials that are likely to be ineffective, leading to unexplained treatment failures and likely delay of appropriate treatment.

In some examples, the method and system of the disclosure can determine the effectiveness of a combination therapy (e.g., two or more antimicrobial agents). This can result in a possibly effective combination therapy to treat microorganisms (e.g., bacteria) for a variety of reasons, such as an infection by bacteria that has been classified as pan-resistant (resistant to all available drugs).

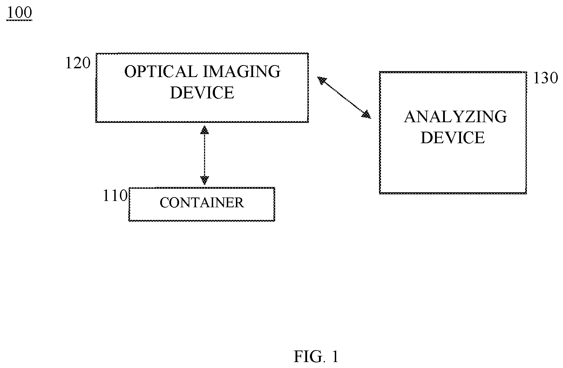

Figure 1 shows a system 100 that can determine a level of susceptibility of a sample to one or more antimicrobial agents using one or more metrics determined from measured surface topography.

In some embodiments, the system 100 may include a culture container 110 holding a clinical sample and one or more antimicrobial agents provided for imaging by an optical imaging device 120. For example, the container 110 may be mounted on a stage.

The optical imaging device 120 may be coupled to an analysis device 130, such as a workstation, personal computer, central processing system, among others. The system 100 can determine a level of susceptibility of a sample to one or more antimicrobial agents provided in the culture container 110 by the analysis device 130 processing the topographic data of one or more regions of the container 110 of the associated growth acquired using the optical imaging system 120.

In some embodiments, the clinical sample (also referred to as "sample") may include a sample collected from any number of sources, including, but not limited to, biological samples (e.g., human samples), environmental samples (e.g., air, agricultural, water, soil, etc.), food samples, among others, or any combination thereof. For example, the biological samples may include but are not limited to bodily fluid such as blood, urine, serum, lymph, saliva, anal and vaginal secretions, skin swabs, perspiration, peritoneal fluid, pleural fluid, effusions, ascites, purulent secretions, lavage fluids, drained fluids, brush cytology specimens, biopsy tissue, explanted medical devices, infected catheters, pus, biofilms, semen, other laboratory specimens from a culture, other types of swabs, among others, or any combination thereof.

In some embodiments, the sample may include one or more microorganisms. The one or more microorganisms may include but is not limited to bacteria, archaebacteria, yeasts, viruses, prions, fungi, algae, protozoa, other pathogens, among others, or any combination thereof.

In some embodiments, the sample may be exposed to one or more antimicrobial agents in the culture medium container 110. The agents may include one or more of antimicrobial agents or one or more agents with antimicrobial activity that suppress or limit the growth or viability of microorganisms. In some embodiments, the one or more antimicrobial agents may include but are not limited to one or more antibiotic agents or one or more agents with antibiotic activity that suppress or inhibit growth or viability of agents. The agents may include but are not limited to chemical compounds, ultraviolet light, radiation, heating, microwaves, etc. In some embodiments, the culture medium container 110 may also hold one or more of predetermined concentrations of one or more antimicrobial agents.

In some embodiments, the culture container 110 may include a culture plate. In some embodiments, the plate may include one or more sites (also "culture" site(s)) for analyzing susceptibility of a sample to one or more antimicrobial agents. The one or more sites may include but are not limited one or more wells or chambers. In some embodiments, each site may include a culture medium. The culture medium may be any known tissue or cell culture liquid media, solid media, among others, or a combination thereof. For example, the culture medium may include but is not limited to an agar-based media, such as an agar pad, polyacrylamide gel, other solid and/or liquid media, among others, or a combination thereof.

In some embodiments, the culture medium of one or more sites may be configured to hold a predetermined concentration of one or more antimicrobial agents. In some embodiments, the one or more sites of the container may include one or more test sections in which a predetermined concentration of one or more antimicrobial agents may be disposed on the culture medium and/or introduced to the culture medium. In some embodiments, the culture container 110 may include more than one site holding different concentrations of one or more antimicrobial agent(s) and/or one or more different antimicrobial agent(s) so as to determine the susceptibility of the sample to different types/combinations/amounts of antimicrobial agents in a single container 110.

By way of example, one or more sites may include a predetermined concentration of two or more antimicrobial agents. This way, possible effectiveness of a combination therapy may be determined, for example, for a possibly resistant microorganism.

In some embodiments, the culture container 110 may include one or more sites including one or more control sections that includes the culture medium without the one or more antimicrobial agents and/or the sample. By way of example, the control section(s) may include but is not limited to a bare culture medium (e.g., bare agar) and/or the sample without the antimicrobial agent(s).

In some embodiments, one or more sites may include both the control section(s) and one or more test section(s). In some embodiments, the test section(s) and the control section(s) of each site and/or container may include the same culture medium. That way, the control section(s) of the culture container 110 may be used for calibration and/or correction of the topographic data acquired for the sample disposed in the test section(s) of the culture site. In some embodiments, the container 110 may include the control section(s) by itself in the one or more sites and the remaining sites may be only for the test section(s).

In some embodiments, the sample provided in the culture container 110 may be inoculated and incubated for a period of time.

In some embodiments, the optical imaging system 120 may acquire images of the surface topography of one or more regions of each site. In some embodiments, the optical imaging system 120 may be an interferometry system configured to measure surface topography, such as, but not limited to, white light interferometers. In some embodiments, the optical imaging system 120 may be an optical microscope, such as but not limited to differential interference contrast (DIC). In some embodiments, the optical imaging system 120 may be configured to acquire topographic data of one or more regions of each site. The topographic data of one or more regions of each site may include one or more portions of the test section, one or more portions of the control section, among others, or a combination thereof. The topographic data may include a height value for each pixel disposed in the one or more regions. In some embodiments, the height value may be determined using the intensity of the light detected by the optical imaging system 130. This way, the optical imaging system can measure the biofilm surface topography of the sample.

In some embodiments, the analyzing device 130 may use the topographic data (e.g., topographic surface properties or topographic surface profile) to determine or quantify susceptibility of the microorganism to one or more antimicrobial agents provided along with the sample in the container 110. For example, the analyzing device 130 may determine one or more metrics using the measured surface topography of the sample (e.g., each test section), e.g., the topographic data acquired by the optical imaging system 120. By way of example, the height values of the pixels can be used by the system to determine one or more metrics associated with growth of that sample. The one or more metrics may relate to the degree of the microorganism growth at one or more regions of the test section of a site. This way, the one or more metrics may indicate a quantification and/or a qualification of the growth of the microorganism(s) associated with that site.

In some embodiments, "growth" may include any measurable change in the population of the microorganism of the sample provided in the culture container 120. The term "growth" can be used to describe any change, including but not limited to static growth (i.e., a lack of growth or neutral growth), where there may be no measurable change, or no net change, in a measured value of an attribute of a microorganism; negative growth (i.e., necrosis, apoptosis, and/or autophagic cell death) where there may be a reduction in a measured value of an attribute of a microorganism; and positive growth (i.e., growing) where there is an increase in an attribute of a microorganism.

In some embodiments, the one or more metrics may include one or more of volumetric metrics, geometric metrics, curvature metrics, distribution metrics, spatial correlation metrics, among others, or a combination thereof. By way of example, the one or more volumetric metrics for each sample may include a total volume of one or more regions (of the test section), entire test section, among others, or a combination thereof; the one or more geometric metrics may include curvature for the one or more regions and/or entire test section, slope for the one or more regions and/or entire test section, among others, or a combination thereof; the one or more distribution metrics may include skewness for the one or more regions and/or entire test section, kurtosis for the one or more regions and/or entire test section, among others, or a combination thereof; among others; or a combination thereof.

Using the one or more metrics, the system can further determine one or more indices indicating a level of susceptibility, from one or more levels of susceptibility, of (the one or more microorganisms of) the sample to the one or more antimicrobial agents provided at each site. By using a culture container with different types/combinations/amounts of antimicrobial agent(s) disposed at different sites, the system may determine an index of the sample at each site for each type/combination/amount of antimicrobial agent(s) at the respective site.

In some embodiments, the one or more indices may include one or more of a qualitative index, a quantitative index, among others, or any combination thereof. In some embodiments, the qualitative index may be a qualitative category. In some embodiments, the one or more qualitative categories may include phenotypes, such as susceptible, resistant, heteroresistant, among others, or any combination thereof. By way of example, the one or more levels may include one or more degrees associated with each phenotype (e.g., susceptible, resistant, and/or heteroresistant). For example, the one or more qualitative indices having one or more levels may include but is not limited to resistant, high heteroresistant, low heteroresistant, and susceptible.

**In** these examples, "susceptible" can mean that one or more antimicrobial agents could have an inhibitory effect on the growth of microorganism(s) or a lethal effect on the microorganism(s) included in the sample. Identification of "susceptibility," for example, using the systems and methods described herein, may provide information that may be useful to a clinician's decision regarding antimicrobial agent therapy for a patient. "Resistant" can mean that microorganism(s) included in the sample would not be substantially affected by one or more antimicrobial agents. For example, resistance may be identified by determining that a microorganism's growth is not substantially affected by one or more antimicrobial agents provided at that site. "Heteroresistant" can mean that the microorganism(s) included in the sample may be both susceptible and resistant. In some examples, low heteroresistant may be classified as susceptible by conventional susceptibility testing and high heteroresistant may be classified as resistant by conventional susceptibility testing.

In some embodiments, the one or more indices may include a quantitative index (e.g., value). The quantitative index may be a quantitative value. The value may include but is not limited to a number of resistant cells per million, a fraction of resistant cells, growth rate absent antimicrobial agent, among others, or any combination thereof. For example, for the fraction of resistant cells, the quantitative value may be an absolute value of a logarithm (e.g., log base 10, log base 2, natural log, etc.). By way of example, if the quantitative value corresponds to the absolute value of logarithm, base 10, of the fraction of resistant cells, the quantitative index for a fraction of resistant cells of 1/1000000 would be 6, the quantitative index for a fraction of resistant cells of 1/100 would be 2, etc.

In some embodiments, the quantitative value for a site may be compared to one or more thresholds to determine a qualitative category (e.g., susceptible, resistant, heteroresistant, etc.) associated with that state. For example, the thresholds associated with each phenotype (e.g., susceptible phenotypes, heteroresistant phenotypes, and/or resistant phenotypes) may vary based on bacterial species, strain, antibiotic, and other factors relevant for the patient (e.g., age, underlying conditions, etc.).

In some embodiments, the one or more indices for each site may be based on a biophysical relationship between the levels of susceptibility and the one or more metrics. The one or more indices may be determined using, for example, control-based methods, numerical-based methods, statistical-based methods, empirical-based methods, machine-learning based methods, analytical-based methods, computational-based methods, image analytical-based methods, among others, or a combination thereof. For example, the machine-learning based methods may include classifiers trained on topographic data, maps and/or profiles and associated susceptibility (quantitative and/or qualitative) index; the one or more metrics and associated susceptibility (quantitative and/or qualitative) index; among others, or a combination thereof.

In some embodiments, the analysis device 130 may generate and output an analysis report for the one or more sites. The analysis report may include one or more indices for one or more sites. For each site, the analysis report may include one or more indices indicating level of susceptibility of the sample to the one or more antimicrobial agents tested in each site. For example, the analysis report may include at least one quantitative index and/or qualitative index for each site. In another example, the report may additionally include classifying information regarding any microorganisms cultured, the concentration of the microorganisms, growth rate of microorganisms cultured, among others, or a combination thereof.

In some embodiments, the device 120 and/or the device 130 may be disposed within the same device or otherwise have connectivity via a communication network. By way of example, the communication network of system 100 can include one or more networks such as a data network, a wireless network, a telephony network, or any combination thereof. The data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fiber-optic network, and the like, NFC/RFID, RF memory tags, touch-distance radios, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks, code division multiple access (CDMA), wideband code division multiple access (WCDMA), wireless fidelity (WiFi), wireless LAN (WLAN), Bluetooth^{®}, Internet Protocol (IP) data casting, satellite, mobile ad-hoc network (MANET), and the like, or any combination thereof.

Although the systems/devices of the system 100 are shown as being directly connected, the systems/devices may be indirectly connected to one or more of the other systems/devices of the system 100. In some embodiments, a system/device may be only directly connected to one or more of the other systems/devices of the system 100.

It is also to be understood that the system 100 may omit any of the devices illustrated and/or may include additional systems and/or devices not shown. It is also to be understood that more than one device and/or system may be part of the system 100 although one of each device and/or system is illustrated in the system 100. It is further to be understood that each of the plurality of devices and/or systems may be different or may be the same. For example, one or more of the devices of the devices may be hosted at any of the other devices.

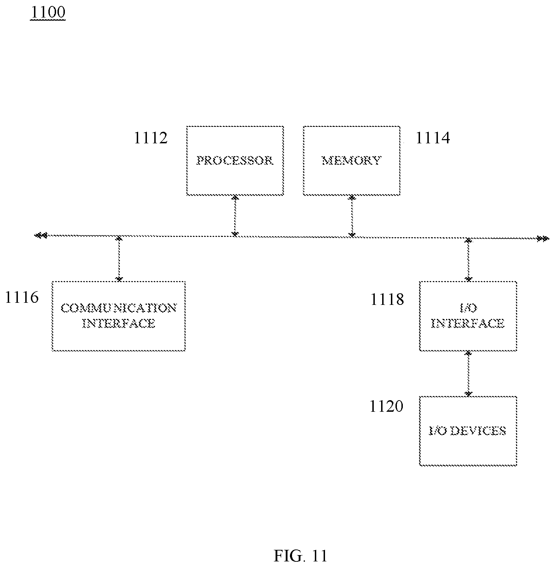

In some embodiments, any of the devices of the system 100, for example, the device 130, may include a non-transitory computer-readable medium storing program instructions thereon that is operable on a user device. A user device may be any type of mobile terminal, fixed terminal, or portable terminal including a mobile handset, station, unit, device, multimedia computer, multimedia tablet, Internet node, communicator, desktop computer, laptop computer, notebook computer, netbook computer, tablet computer, personal communication system (PCS) device, wearable computer (e.g., smart watch), or any combination thereof, including the accessories and peripherals of these devices, or any combination thereof. Figure 11 shows an example of a user device.

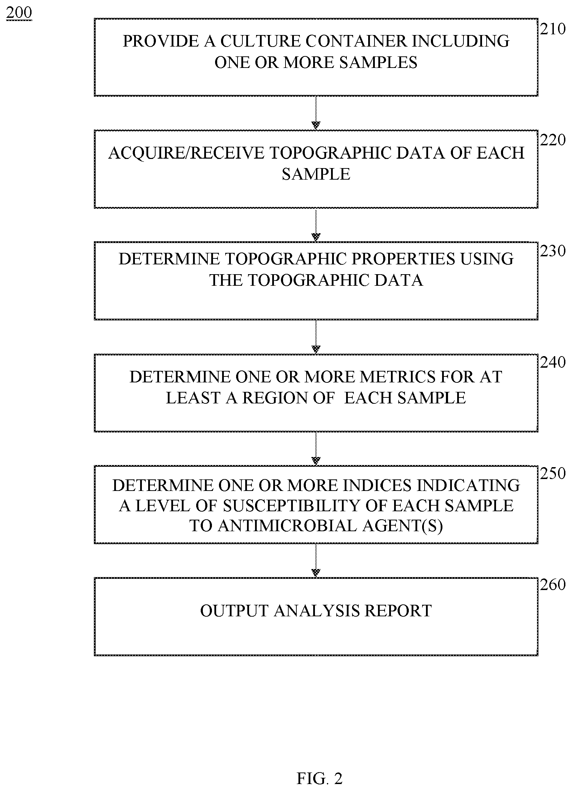

Figure 2 shows a method 200 of determining one or more indices indicating antimicrobial resistance of a sample using one or more topographic surface profiles or their properties according to embodiments. Unless stated otherwise as apparent from the following discussion, it will be appreciated that terms such as "encoding," "generating," "determining," "displaying," "obtaining," "applying," "processing," "computing," "selecting," "receiving," "detecting," "classifying," "calculating," "quantifying," "outputting," "acquiring," "analyzing," "retrieving," "inputting," "assessing," "performing," or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. The system for carrying out the embodiments of the methods disclosed herein is not limited to the systems shown in Figures 1 and 11. Other systems may also be used.

The methods of the disclosure are not limited to the steps described herein. The steps may be individually modified or omitted, as well as additional steps may be added. It will be also understood that at least some of the steps may be performed in parallel.

As shown in Figure 2, the method 200 may include a step 210 of providing one or more prepared samples disposed in a culture medium container to the system to be analyzed according to some embodiments. In some embodiments, each prepared sample may be provided in a site or chamber of the culture medium. For example, the sample may be prepared using any available methods, such as inoculation and incubation. By way of example, for testing antibiotic resistance, a sample may be prepared by (i) placing a small drop of the sample that includes one or more microorganisms on an agar pad that includes a predetermined concentration of one or more antibiotics disposed within a site; and (ii) incubating the sample for a period of time (e.g., 0.5-3 hours).

The method 200 may include a step 220 of acquiring and/or receiving topographic data of a region of each sample/site of the container by/from an optical imaging system. By way of example, an interferometer may acquire images of one or more regions of each site so as to measure the associated topography. In one example, if the container includes more than one site, the optical imaging system may acquire topographic data of a region that includes the control section for one site; and topographic data of a region that includes the test section of the same size and location for each site. In another example, if the container includes more than one site, the optical imaging system may acquire topographic data of regions of the same size and location that includes the testing and control sections for each site. This can result in a map of the raw topography for the one or more regions for each site. The raw topographic data may include pixels which represent different positions in the topographic image or map and each pixel may include a height for that location.

In some embodiments, the method 200 may include a step 230 of determining one or more topographic surface properties or profiles for one or more regions of each site that includes the test section using the topographic data associated with that sample/site. In some embodiments, the topographic profile for one or more regions of the test section of one or more sites may be calibrated using the topographic data for one or more regions of the control section without antimicrobial agent(s) and the sample (e.g., bare culture medium (e.g., bare agar)) of one or more sites of the container to determine a pure topographic profile. For example, the topographic profile for the test section for each site may be calibrated with topographic data (or profile) for the control section for that site and/or for another site of the container using any available methods to determine the pure topographic profile. In some embodiments, the one or more topographic surface properties or profiles for a region/site (e.g., pure topographic profile) may also be used to train a machine-learning model (step 250). In some embodiments, the topographic surface properties (or profiles), including the pure topographic profile, may be represented in a form of a topographic (surface) image or map.

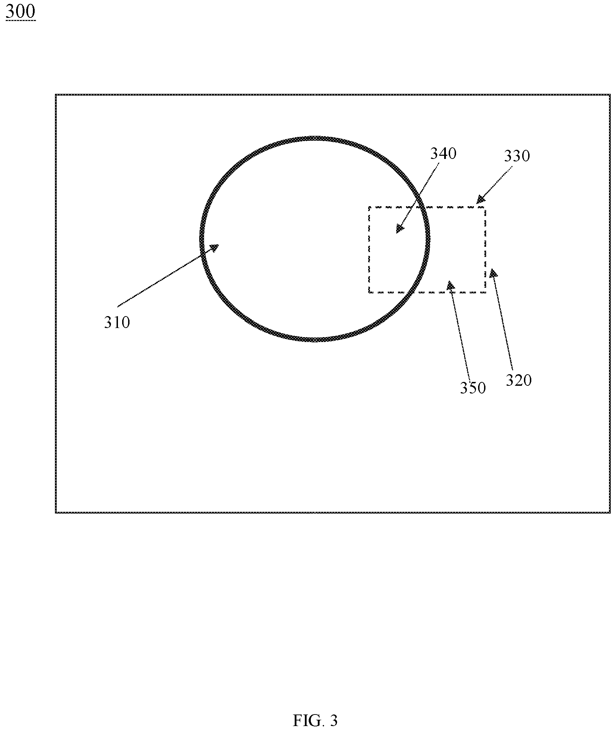

Figure 3 shows an example 300 of a site that includes a section 310 and a section 320. The section 310 may be a test section in which a sample is disposed along with a predetermined concentration of one or more antimicrobial agents or a control section in which the sample is disposed without a one or more antimicrobial agents. The site may also include a control section 320 in which the culture medium is disposed without the sample and the one or more antimicrobial agents (e.g., bare agar). In this example, the optical imaging system 110 acquired topographic data for region 330 that includes a region or portion 340 of the section 310 and a region or portion 350 of the section 320. In this example, the raw topographic data for the region 350 may be used to calibrate the raw topographic data for the region 340 resulting in a (calibrated) topographic data, such as topographic image or map, of a region or the entire sample disposed section 310.

By way of example, in this step, the raw topographic data can be processed to remove the graduation resulting from the culture medium included in the container and/or from the optical imaging device such that a height value for a pixel location of 0 can represent no microorganism (e.g., bacteria) at that location. For example, the step 230 may include fitting a plane to the surface of the region(s) of the control section of one or more sites, extrapolating the fit plane across the topographic map/profile for the other regions (e.g., test section(s)) of that site, other sites, and/or each site. The step 230 may further include determining one or more topographic properties (or profiles) in a form of a topographic map of the test section of each site by subtracting that best-fit plane from the topographic map or profile of each site. A region or subsection of the test section of that site may be considered to be the pure topographic profile for that site. This way, the height values representing growth of the sample disposed within the test section(s) of each site may be determined.

Next, the method 200 may include a step 240 of determining one or more metrics for each site including a test section using the topographic surface properties (or profiles) of the corresponding region(s) and/or entire section. In some embodiments, the step 240 may include determining one or more metrics for a site including a control section that includes the sample without antimicrobial agent(s) using the one or more topographic surface properties (or profiles) (or topographic image/map) of the corresponding region(s) and/or entire section.

In some embodiments, the one or more metrics may include one or more of volumetric metrics, geometric metrics, curvature metrics, distribution metrics, spatial correlation metrics, machine identified or derived metrics, among others, or a combination thereof. By way of example, the one or more volumetric metrics may include a total volume of one or more regions (of the test section) at the site, entire test section at that site, among others, or a combination thereof; the one or more geometric metrics may include curvature for the one or more regions and/or entire test section, slope for the one or more regions and/or entire test section, among others, or a combination thereof; the one or more distribution metrics may include skewness for the one or more regions and/or entire test section of that site, variance for the one or more regions and/or entire test section of that site, kurtosis for the one or more regions and/or entire test section of that site, among others, or a combination thereof; among others; or a combination thereof. The one or more metrics may be determined using any available methods and are not limited to those described. For example, the method(s) may include but is not limited to control-based methods, numerical-based methods, statistical-based methods, empirical-based methods, machine-learning based methods, analytical-based methods, computational-based methods, image analytical-based methods, among others, or a combination.

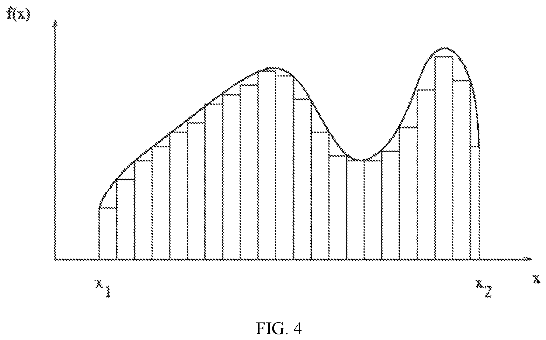

For example, total volume for the test section of a section can be determined using numerical integration. Figure 4 shows an example of a numerical integration according to embodiments. In this example, the volume may be determined by treating each pixel as a rectangular box with cross-sectional area equal to the pixel size squared and the height of the box equal to the measured height at that pixel as shown in Figure 4. In Figure 4, the integral of the curve yields the area under the curve. It can be approximated by dividing the curve into many rectangles and estimating the area as the sum of the area of the rectangles. In other examples, volume can be calculated by numerically integrating over the topographic map (or profile) using one of many numerical approaches, such as Simpson's rule.

In another example, for one or more geometric metrics using slope, the step 240 may include determining a first spatial map of the slope by subtracting the height of given pixel from the heights of its neighboring pixels. This first spatial map may relate to slope. To determine curvature, the step 240 may include generating a map of the curvature (i.e., the slope of slopes) by generating a second spatial map of the first spatial map by subtracting the height of given pixel from the heights of its neighboring pixels, and then by generating a third spatial map of the second spatial map by subtracting the height of given pixel from the heights of its neighboring pixels.

In another example, for kurtosis, the step 240 may include determining the average height of the region, selecting each pixel and calculating its height minus the average height, and then cubing it: (hₚᵢₓₑₗ - h_{avg})³. This value may then be averaged over all pixels and divided by the standard deviation cubed to determine kurtosis for that region/site.

In another example, for one or more metrics related to spatial correlation functions, the one or more metrics can relate to a distance over which a value (e.g., width, height, etc. ) is similar within the region/section. In some embodiments, the spatial correlation functions may determine the number of cells that are growing within the region.

For example, the one or more metrics related to spatial correlation functions may include determining one or more metrics using a height-height correlation function. In this example, the height-height correlation may measure how similar the height is, on average, between two locations separated by a distance r and the one or more metrics may be determined from how this function decays.

Next, the method 200 may include a step 250 of determining one or more indices representing or indicating a level associated with susceptibility of the sample to one or more concentrations of one or more antimicrobials for one or more regions/sites using one or more metrics associated with that region/site. For example, the one or more indices for each site may include one or more qualitative and/or quantitative indices. Each index may be determined using any known or available biophysical relationship between the levels of susceptibility and one or more metrics. By way of example, the index may be determined using, for example, one or more of control-based methods, numerical-based methods, statistical-based methods, empirical-based methods, machine-learning based methods, analytical-based methods, computational-based methods, image analytical-based methods, among others, or a combination thereof.

For example, the machine-learning based methods may include but is not limited to Bayes classifier, support vector machine (SVM), linear discriminant functions, Fisher's linear discriminant, C4.6 algorithm tree, K-nearest neighbor, weighted K-nearest neighbor, Hierarchical clustering algorithm, a learning algorithm that incorporates an ensemble classifier that uses the methods developed by Breiman and Cutler, hidden Markov model, Gaussian mixture model (GMM), K-mean clustering algorithm, Ward's clustering algorithm, minimum least squares, neural network algorithms, logistic regression, among others, or a combination thereof.

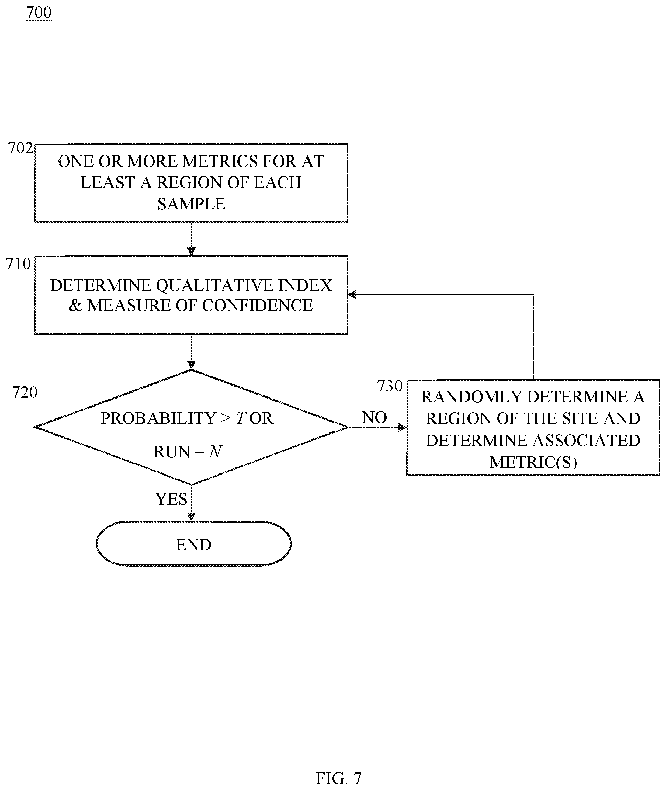

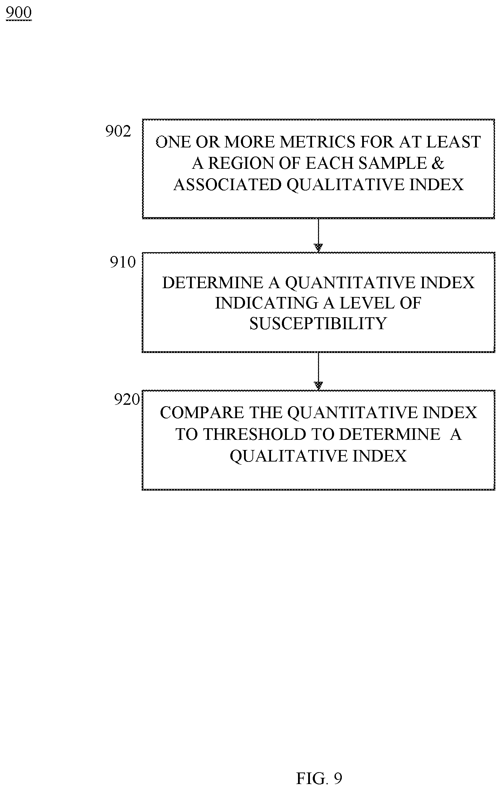

By way of example, one or more of the classifiers may be trained or developed using topographic surface properties (e.g., maps or profiles), one or more metrics and associated qualitative and/or quantitative susceptibility indices, among others, or a combination thereof. In another example, the step 250 may also include a measure of confidence (e.g., probability that the determined index is correct) of the determination of the index by the one or more classifiers. The training and/or determination of the measure of confidence may be performed and/or determined using any available methods. In another example, the one or more classifiers may include more than one classifier. For example, one or more of the classifiers may determine a qualitative index for a site and another one or more of the classifiers may determine a quantitative index for that site using the determined qualitative index. For example, Figures 7 and 9 show examples of methods determining one or more qualitative indices and one or more quantitative indices, respectively, using trained classifiers according to embodiments.

For example, for total volume, an index for a region may be determined by comparing the volume determined for each site/test section including the sample and one or more antimicrobial agents to the volume determined for the control section that includes the sample without one or more antimicrobial agents. These volume measurements may then be analyzed using one or more biophysical models. For example, a biophysical model may relate an index indicating a level of susceptibility to the ratio of the volumes with and without antibiotics: *hs = V*/*V*₁, where V is the volume of the population, *V*₁ is the volume of a population absent any antimicrobial agents (e.g., antibiotics), and *hs* is a value representing the index. By way of example, if *hs <* 10⁻⁶, the sample may be considered to be susceptible to the one or more antimicrobial agents disposed therein. If *hs >* 0.01 for a test section, the sample may be considered to resistant to the one or more antimicrobial agents disposed therein. If the value is between those thresholds (limits), the sample may be considered to heteroresistant to the one or more antimicrobial agents disposed therein.

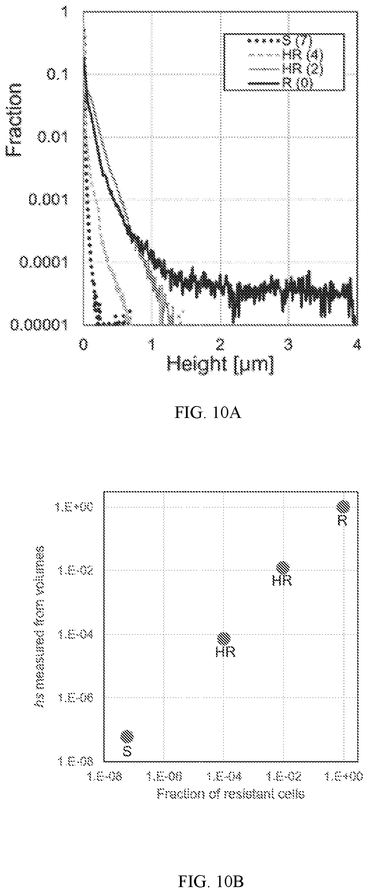

For example, Figure 10A shows an example of relationship of the susceptibility index using volume according to embodiments. In this example, *hs,* the susceptibility index, extracted from volume measurements, versus the fraction of resistant cells, as measured by population analysis profiling is plotted. The susceptibility index, which is indicated by phenotype, for each data point is indicated by "R" for resistance, "HR" for heteroresistance, or "S" for susceptible and is provided underneath the associated data point.

In another example, for curvature, the curvature determined for a test section/site may be compared to a threshold and/or a curvature determined for the control section that includes the sample without one or more antimicrobial agents to determine the index. For example, if the curvature for the test section/site is much smaller than the curvature of the control section/site that includes the sample without one or more antimicrobial agents (such as 50% smaller), the populations have a lot of cell death, then the sample may be considered susceptible to the one or more antimicrobial agents tested in that site. If the sample curvature similar to that of the curvature of the control section that includes the sample without one or more antimicrobial agents (such as no more than 10% smaller), the population does not have a lot of cell death, then the sample may be considered resistant to the one or more antimicrobial agents tested in that site. If the sample curvature is between the upper thresholds (above limits), such as greater than 50%, but less than 90% of the curvature of the control section that includes the sample without one or more antimicrobial agents, then the sample may be considered heteroresistant to the one or more antimicrobial agents tested in that site. Populations with no growth or death (e.g., a population in the presence of a bacteriostatic drug) could have the curvature that resulted during inoculation, for example, if the sample is susceptible or heteroresistant to the one or more antimicrobial agents tested in that site. Thus, curvature can distinguish populations with similar sizes but different amounts of death and reproduction.

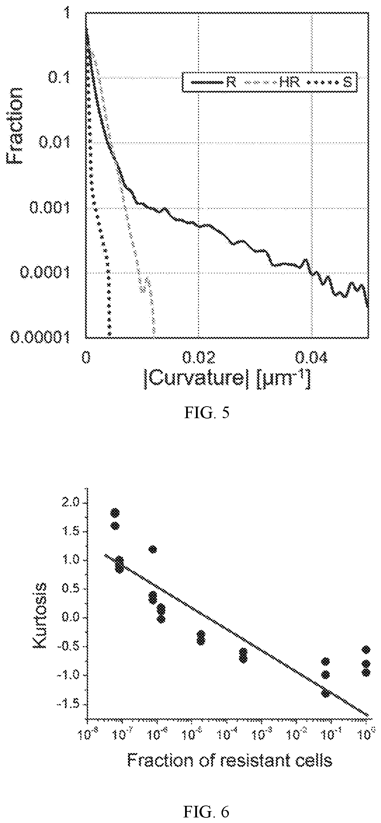

In another example, empirical methods may be used to determine the index associated with the curvature. For example, Figure 5 shows an example of histograms of curvature for regions having *Klebsiella pneumonia* isolates with different qualitative index (susceptibilities) to colistin after incubation for 90 minutes. As shown, the histograms can demonstrate the differences between indices indicating susceptible, heteroresistant, and resistant phenotypes.

In another example, for kurtosis, empirical methods may be used to determine the associated index. By way of example, if the kurtosis is above a first threshold (e.g., 0), the sample may be considered to be susceptible to the one or more one or more antimicrobial agents tested in that site; and if the kurtosis is below a second threshold (-0.76), the sample may be considered to be resistant to the one or more one or more antimicrobial agents tested in that site. In a further example, if the kurtosis value is between those thresholds, the sample may be considered to be heteroresistant to the one or more one or more antimicrobial agents tested in that site. For example, if the thresholds include the second threshold for resistant phenotypes, the second threshold value may substantially correspond to and/or include the kurtosis value for the control section that includes the sample without antimicrobial agent(s).

Figure 6 shows an example of surface topographies of eight different CF PA isolates with different qualitative index (susceptibilities) to colistin that were measured after 90 minute incubation, with three replicates of each. In this example, the kurtosis exhibits a strong correlation with the log of the fraction of resistant cells (red line; R = 0.85).

In some embodiments, the method 200 may include a step 260 of outputting and/or generating an analysis report for the one or more sites. For example, the analysis device 130 may generate and/or output an analysis report for the one or more sites. In some embodiments, the analysis report may include the one or more indices for one or more sites. For each site, the analysis report may include one or more indices (qualitative and/or quantitative), indicating level of susceptibility of the microorganisms to the one or more concentrations of the one or more antimicrobial agents tested, the measure of confidence associated with the determined index (level of susceptibility), recommendations for treatment, topographic images, among others, or a combination thereof. In another example, the report may additionally or alternatively include classifying information regarding any microorganisms cultured, the concentration of the microorganisms, among others, or a combination thereof. The outputting may include but is not limited to displaying the analysis report and/or related information, among others, or any combination thereof.

Figure 7 shows an example of a method 700 of determining a qualitative index indicating a level of susceptibility of a sample to one or more antimicrobial agents using machine learning classifier (step 250) according to embodiments.

In some embodiments, the method 700 may include a step 710 of determining an index representing or indicating a level associated with susceptibility of the sample to one or more concentrations of one or more antimicrobials for one or more regions/sites using a (first) trained machine-learning classifier. In some embodiments, the machine learning classifier may be trained using one or more metrics, one or more topographic profile or properties (e.g., entire pure and/or raw topographic map of the test section for a site), associated index (e.g., phenotype measured via Population Analysis Profiling), among others, or a combination thereof.

In some embodiments, the step 710 may include determining a qualitative (e.g., resistant/heteroresistant/susceptible) index by classifying the one or more metrics associated with that region/site 702 (determined in step 240) and associated topographic profile (determined in step 230) using the trained machine-learning classifier. For example, the one or more metrics may include curvature and the topographic profile may include the pure topographic profile. In this example, the trained machine-learning classifier may also be used to determine a measure of confidence (e.g., probability that the determined index is correct) associated with the determined index.

In some embodiments, the method 700 may include a step 720 of comparing the measure of confidence associated with the determined index (e.g., heteroresistant) to a threshold (T) (for example, 90%). If the measure of confidence is below the threshold, the method 700 may include repeating the step 720 using a randomly sampled region or subsection of site and its associated topography and metrics. For example, the region/subsection of the test section of the site may be randomly selected and the associated metrics (step 240) may be determined. The randomly selected region/subsection and associated metrics may then be inputted in the step 710. In some embodiments, the step 710 may be repeated, for example, until the measure of confidence for a subsection/region of the site is higher than the threshold and/or the number of runs or iterations is at the limit (N).

After the measure of confidence is higher than the threshold and/or the number of runs is at the limit, the method 700 may end. In some embodiments, the qualitative index may be used to determine a quantitative index for that region/site (see, e.g., Figure 9). In some embodiments, the report may additionally and/or alternatively be generated (step 260) using the determined qualitative index (step 710). In some embodiments, the report may include the index and associated measure of confidence for each run performed in step 710, for example, when the measure of confidence does not exceed the threshold. In some embodiments, the report may additionally and/or alternatively include the corresponding quantitative index (Figure 9) and/or associated qualitative index (Figure 9) using the measure of confidence for the determined qualitative index (Step 720).

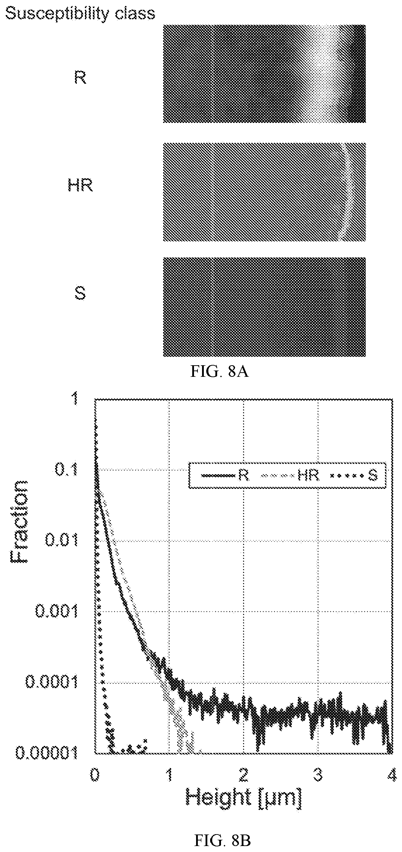

Figures 8A and 8B show examples of topographic maps from Carbapenem-resistant *Klebsiella* isolates with different qualitative index (susceptibilities) to colistin and the resulting determined qualitative indices, respectively. Figure 8A shows three example topographic maps from Carbapenem-resistant *Klebsiella* isolates with qualitative indexes, resistant (R), heteroresistant (HR), and susceptible (S), to colistin. Figure 8B shows a graphical comparison of the determined qualitative indices and related metrics. Figure 8B shows of the relationship between the heights of the *Klebsiella* populations and the determined qualitative index. As shown, the heights of each location in *Klebsiella* populations demonstrate that even low frequency heteroresistance (in this example, ~1 resistant cell per one million total cells) differ significantly from susceptible populations. These heights were determined by counting the number of pixels that correspond to heights between 0 and 4.0 microns, in bins 10 nm wide (i.e., 51 nm, 55 nm, and 59 nm all count in the same bin, 61 nm would be in the next bin).

Figure 9 shows an example of a method 900 of determining a quantitative index indicating a level of susceptibility of a sample to one or more antimicrobial agents using a (second) trained machine learning classifier and the associated qualitative index (Figure 7) (step 250) according to embodiments. In some embodiments, Figure 9 may determine quantitative index from which a qualitative index may be determined without performing the method 700.

In some embodiments, the method 900 may include a step 910 of determining a quantitative index representing or indicating a level associated with susceptibility of the sample to one or more concentrations of one or more antimicrobials for one or more regions/sites using a (second) trained machine-learning classifier. In this example, the method 900 may use the qualitative index and associated topographic data and/or measure(s) (step 902), for example, determined/used in the method 700. For example, in some embodiments, a qualitative index having a measure of confidence above a threshold (e.g., 90% probability) and the associated topographic data for a region/site may be used to determine the corresponding quantitative index for that region/site. In other embodiments, a quantitative index may be determined for each region/site for which a qualitative index was determined (Figure 7). In some embodiments, the qualitative index (step 902) may be omitted and the quantitative index may be determined using only the topographic data and/or metrics for a site.

In some embodiments, the machine learning classifier may be trained using one or more metrics, one or more topographic profile or properties (e.g., entire raw and/or pure topographic map of the test section), known fraction of resistant cells (e.g., measured via Population Analysis Profiling), among others, or a combination thereof.

In some embodiments, the step 910 may include determining a quantitative index by classifying the one or more metrics (902) associated with that region/site (determined in step 240) and associated topographic profile (determined in step 220) for each site using the trained machine-learning classifier. In some embodiments, the quantitative index may be determined using the one or more metrics 902 associated with that region/site (determined in step 240) and associated topographic profile (determined in step 220) for which a qualitative index has a measure of confidence higher than a threshold (method 700) using the trained machine-learning classifier. For example, if a region was determined to have a qualitative index (e.g., phenotype such as HR) and a measure of confidence (e.g., 90% probability), the associated topography profile and the one or more metrics (curvature) may be used by the (second) machine-learning classifier to determine a fraction of resistant cells (e.g., quantitative index).

By way of example, the fraction of resistant cells may be provided in order of magnitude of the fraction. Higher fractions of resistant cells can be generally proportionally associated with taller topographies, as well as a systematic shift to higher curvatures. For example, if the one resistant cell per one million total cells fraction is determined to be the fraction 10⁻⁶, the classifier may determine the quantitative index to be a "6," order of magnitude of the fraction.

In some embodiments, the method 900 may include a step 920 of comparing the determined quantitative index to a threshold to determine a qualitative index. In some embodiments, the qualitative index may change and/or may be same as the qualitative index determined in Figure 7. For example, if the threshold is set at a fraction of resistant cells of 1/100,000 for susceptible (S) and only one resistant cell per one million cells (1/1,000,000) was determined in step 910, then the qualitative index would be determined to be "S."

In some embodiments, the method 900 may end after the step 920. In some embodiments, the quantitative index (e.g., 6) determined in step 910 for each region/site may be then reported (in step 260) along with the associated qualitative index (e.g., S) determined based on the quantitative index in step 920, the associated qualitative index determined in Figure 7, among others, or any combination thereof. By way of example, the report may indicate a quantitative index of "6" and the associated qualitative index of susceptible "S" as "S6." In some embodiments, only the quantitative index and/or qualitative index determined in Figure 9 may be reported if the associated qualitative index determined in Figure 7 has a measure of confidence below a threshold.

Figure 10A shows an example of a height histogram determined for Carbapenem-resistant Klebsiella isolates. Topographic maps were measured; heights were rounded to the nearest 0.01 microns and then counted in this histogram. As shown, the height histogram can be used to determine a qualitative phenotype (R/HR/S), for example, using the method as shown and described in Figure 7. In some embodiments, the heights here can also be used determine a quantitative index, for example, using the method as shown and described in Figure 9.

Figure 10B shows an example of quantitative index and associated quantitative-based qualitative index determined using the height histogram determined in Figure 10A and the method shown and described in Figure 9 according to embodiments. In this example, *hs,* the susceptibility index, extracted from volume measurements, versus the fraction of resistant cells, as measured by population analysis profiling is plotted. The susceptibility index, which is indicated by phenotype, for each data point is indicated by "R" for resistance, "HR" for heteroresistance, or "S" for susceptible and is provided underneath the associated data point.

One or more of the devices and/or systems of the system 100 may be and/or include a computer system and/or device. Figure 11 is a block diagram showing an example of a computer system 1100. The modules of the computer system 1100 may be included in at least some of the systems and/or modules, as well as other devices and/or systems of the system 100.

The system for carrying out the embodiments of the methods disclosed herein is not limited to the systems shown in Figures 1 and 11. Other systems may also be used. It is also to be understood that the system 1100 may omit any of the modules illustrated and/or may include additional modules not shown.

The system 1100 shown in Figure 11 may include any number of modules that communicate with each other through electrical or data connections (not shown). In some embodiments, the modules may be connected via any network (e.g., wired network, wireless network, or any combination thereof).

The system 1100 may be a computing system, such as a workstation, computer, or the like. The system 1100 may include one or more processors 1112. The processor(s) 1112 may include one or more processing units, which may be any known processor or a microprocessor. For example, the processor(s) may include any known central processing unit (CPU), graphical processing unit (GPU) (e.g., capable of efficient arithmetic on large matrices encountered in deep learning models/classifiers), among others, or any combination thereof. The processor(s) 1112 may be coupled directly or indirectly to one or more computer - readable storage media (e.g., memory) 1114. The memory 1114 may include random access memory (RAM), read only memory (ROM), disk drive, tape drive, etc., or any combinations thereof. The memory 1114 may be configured to store programs and data, including data structures. In some embodiments, the memory 1114 may also include a frame buffer for storing data arrays.

In some embodiments, another computer system may assume the data analysis, image processing, or other functions of the processor(s) 1112. In response to commands received from an input device, the programs or data stored in the memory 1114 may be archived in long term storage or may be further processed by the processor and presented on a display.

In some embodiments, the system 800 may include a communication interface 1116 configured to conduct receiving and transmitting of data between other modules on the system and/or network. The communication interface 816 may be a wired and/or wireless interface, a switched circuit wireless interface, a network of data processing devices, such as LAN, WAN, the internet, or any combination thereof. The communication interface may be configured to execute various communication protocols, such as Bluetooth, wireless, and Ethernet, in order to establish and maintain communication with at least another module on the network.

In some embodiments, the system 1110 may include an input/output interface 1118 configured for receiving information from one or more input devices 1120 (e.g., a keyboard, a mouse, and the like) and/or conveying information to one or more output devices 1120 (e.g., a printer, a CD writer, a DVD writer, portable flash memory, etc.). In some embodiments, the one or more input devices 1120 may be configured to control, for example, the generation of the management plan and/or prompt, the display of the management plan and/or prompt on a display, the printing of the management plan and/or prompt by a printer interface, the transmission of a management plan and/or prompt, among other things.

In some embodiments, the disclosed methods (e.g., Figures 2, 7 and 9) may be implemented using software applications that are stored in a memory and executed by the one or more processors (e.g., CPU and/or GPU) provided on the system 100. In some embodiments, the disclosed methods may be implemented using software applications that are stored in memories and executed by the one or more processors distributed across the system.

As such, any of the systems and/or modules of the system 100 may be a general purpose computer system, such as system 1100, that becomes a specific purpose computer system when executing the routines and methods of the disclosure. The systems and/or modules of the system 100 may also include an operating system and micro instruction code. The various processes and functions described herein may either be part of the micro instruction code or part of the application program or routine (or any combination thereof) that is executed via the operating system.

If written in a programming language conforming to a recognized standard, sequences of instructions designed to implement the methods may be compiled for execution on a variety of hardware systems and for interface to a variety of operating systems. In addition, embodiments are not described with reference to any particular programming language. It will be appreciated that a variety of programming languages may be used to implement embodiments of the disclosure. An example of hardware for performing the described functions is shown in Figures 1 and 11. It is to be further understood that, because some of the constituent system components and method steps depicted in the accompanying figures can be implemented in software, the actual connections between the systems components (or the process steps) may differ depending upon the manner in which the disclosure is programmed. Given the teachings of the disclosure provided herein, one of ordinary skill in the related art will be able to contemplate these and similar implementations or configurations of the disclosure.

While the disclosure has been described in detail with reference to exemplary embodiments, those skilled in the art will appreciate that elements and/or features of different exemplary embodiments may be combined with each other and/or substituted for each other within the scope of this disclosure and appended claims.

## Claims

1. A method of determining a level of susceptibility of a sample to one or more antimicrobials based on topographic surface properties, comprising:
providing a container (110) including one or more sites, the one or more sites including a sample having one or more microorganisms and one or more concentrations of one or more antimicrobial agents;
receiving a topographic surface profile for one or more regions (330, 340, 350) of each site, the one or more regions including at least one region having the sample and a concentration of the one or more antimicrobial agents;
determining one or more metrics of at least one region of the one or more regions of each site using the topographic surface profile for each site, the one or more metrics including one or more of volumetric metrics, distribution metrics, spatial correlation metrics, among others, or a combination thereof; and
determining one or more indices representing a level of susceptibility of the sample to the concentration of the one or more antimicrobial agents provided in each site using the one or more metrics for the at least one region of each site from one or more indices;
wherein the topographic surface profile includes a height value for each pixel disposed within the associated region; and
the one or more metrics is determined using the height value for each pixel disposed within the associated region.

2. The method of claim 1, wherein the one or more indices includes one or more qualitative indices, the one or more qualitative indices including a first index indicating that the sample is susceptible to the concentration of the one or more antimicrobial agents and a second index indicating that the sample is resistant to the concentration of the one or more antimicrobial agents, optionally wherein the one or more qualitative indices includes a third index indicating that the sample is heteroresistant to the concentration of the one or more antimicrobial agents.

3. The method of either of claims 1 or 2, wherein the one or more antimicrobial agents includes one or more antibiotic agents.

4. The method of any of claims 1-3, wherein each site includes a culture medium, optionally wherein the culture medium includes a solid medium and/or a liquid medium.

5. The method of claim 4, wherein the culture medium includes an agar medium.

6. The method of any of claims 1-5, wherein the determining the one or more metrics uses pixels of the associated region of a topographic image acquired by an optical imaging device.

7. The method of any of claims 1-6, wherein the one or more volumetric metrics includes a total volume of at least one region of each site.

8. The method of any of claims 1-7, wherein the one or more distribution metrics includes kurtosis.

9. The method of any of claims 1-8, wherein the one or more indices is determined based on a biophysical relationship between the one or more metrics and the one or more indices.

10. The method of any of claims 1-9, wherein the one or more sites includes at least one site and/or at least one section that includes the sample without the one or more antimicrobial agents, the method further comprising:
determining one or more metrics of the at least one site and/or the at least one section that includes the sample without the one or more antimicrobial agents, the one or more metrics including one or more of volumetric metrics, distribution metrics, spatial correlation metrics, among others, or a combination thereof;
wherein the determining the one or more indices includes comparing the one or more metrics of the one or more sites including the sample and the one or more antimicrobial agents to the one or more metrics of the at least one site and/or the at least one section that includes the sample without the one or more antimicrobial agents.

11. The method of any of claims 1-10, wherein each site includes (i) one or more test sections, each test section having the sample and a concentration of one or more antimicrobial agents disposed on a culture medium and/or (ii) one or more control sections having the culture medium without the one or more antimicrobial agents.

12. The method of claim 11, wherein the one or more control sections includes one or more control sections having the culture medium without the one or more antimicrobial agents and the sample.

13. The method of claim 12, further comprising:
obtaining raw topographic data of one or more regions of the test section and/or the control section using optical imaging;
calibrating the raw topographic data for the one or more regions of the test section with the raw topographic image data for the one or more regions of the control section; and
generating the topographic profile for one or more regions of the test section of each site, optionally wherein the topographic profile is represented by a topographic map.

14. The method of any of claims 1-13, wherein the one or more indices includes one or more of quantitative and/or qualitative indices, optionally wherein the one or more quantitative indices is determined using one or more qualitative indices and topographic profile associated with each site.

15. A system for determining a level of susceptibility of a sample to one or more antimicrobials based on topographic surface properties, comprising:
at least one processor; and
a memory, the memory having stored thereon computer-executable instructions which, when executed by the at least one processor, cause the at least one processor to perform the steps of:
receiving a topographic surface profile for one or more regions (330, 340, 350) of each site of one or more sites of a container, the one or more sites including a sample having one or more microorganisms and one or more concentrations of one or more antimicrobial agents, the one or more regions including at least one region having the sample and a concentration of the one or more antimicrobial agents;
determining one or more metrics of at least one region of the one or more regions of each site using the topographic surface profile for each site, the one or more metrics including one or more of volumetric metrics, distribution metrics, spatial correlation metrics, among others, or a combination thereof; and
determining one or more indices representing a level of susceptibility of the sample to the concentration of the one or more antimicrobial agents provided in each site using the one or more metrics for the at least one region of each site from one or more indices;
wherein the topographic surface profile includes a height value for each pixel disposed within the associated region; and
the one or more metrics is determined using the height value for each pixel disposed within the associated region.

16. The system of claim 15, further comprising the container.

17. The system of claims 15 or 16, wherein the one or more antimicrobial agents includes one or more antibiotic agents.

18. The system of any of claims 15-17,
wherein each site includes a culture medium,
optionally wherein the culture medium includes a solid medium and/or a liquid medium.

19. The system of any of claims 15-18, wherein the determining the one or more metrics uses pixels of the associated region of a topographic image acquired by an optical imaging device.

20. The system of any claims 15-19, wherein the one or more volumetric metrics includes a total volume of at least one region of each site.

21. The system of any of claims 15-20, wherein the one or more sites includes at least one site and/or at least one section that includes the sample without the one or more antimicrobial agents, wherein the computer-executable instructions further cause the at least one processor to perform the steps of:
determining one or more metrics of the at least one site and/or the at least one section that includes the sample without the one or more antimicrobial agents, the one or more metrics including one or more of volumetric metrics, distribution metrics, spatial correlation metrics, among others, or a combination thereof;
wherein the determining the one or more indices includes comparing the one or more metrics of the one or more sites including the sample and the one or more antimicrobial agents to the one or more metrics of the at least one site and/or the at least one section that includes the sample without the one or more antimicrobial agents.

22. The system of any of claims 15-21,
wherein each site includes (i) one or more test sections, each test section having the sample and a concentration of one or more antimicrobial agents disposed on a culture medium and/or (ii) one or more control sections having the culture medium without the one or more antimicrobial agents;
optionally, wherein the one or more control sections includes one or more control sections having the culture medium without the one or more antimicrobial agents and the sample.

23. The system of claim 22, wherein the computer-executable instructions further cause the at least one processor to perform the steps of:
obtaining raw topographic data of one or more regions of the test section and/or the control section using optical imaging;
calibrating the raw topographic data for the one or more regions of the test section with the raw topographic image data for the one or more regions of the control section; and
generating the topographic profile for one or more regions of the test section of each site, optionally wherein the topographic profile is represented by a topographic map.

24. The system of any of claims 15-23,
wherein the one or more indices includes one or more of quantitative and/or qualitative indices,
optionally wherein the one or more quantitative indices is determined using one or more qualitative indices and topographic profile associated with each site,
optionally, wherein the one or more qualitative indices includes a first index indicating that the sample is susceptible to the concentration of the one or more antimicrobial agents and a second index indicating that the sample is resistant to the concentration of the one or more antimicrobial agents, optionally wherein the one or more qualitative indices includes a third index indicating that the sample is heteroresistant to the concentration of the one or more antimicrobial agents.

## Patentansprüche

1. Verfahren zur Bestimmung eines Grades der Empfindlichkeit einer Probe gegenüber einem oder mehreren antimikrobiellen Mitteln basierend auf topographischen Oberflächeneigenschaften, umfassend:
Bereitstellen eines Behälters (110), der eine oder mehrere Stellen beinhaltet, wobei die eine oder die mehreren Stellen eine Probe mit einem oder mehreren Mikroorganismen und einer oder mehreren Konzentrationen eines oder mehrerer antimikrobieller Mittel beinhalten;
Empfangen eines topographischen Oberflächenprofils für eine oder mehrere Regionen (330, 340, 350) jeder Stelle, wobei die eine oder die mehreren Regionen mindestens eine Region mit der Probe und einer Konzentration des einen oder der mehreren antimikrobiellen Mittel beinhalten;
Bestimmen einer oder mehrerer Metriken mindestens einer Region der einen oder mehreren Regionen jeder Stelle unter Verwendung des topographischen Oberflächenprofils für jede Stelle, wobei die eine oder die mehreren Metriken unter anderem eine oder mehrere von volumetrischen Metriken, Verteilungsmetriken, räumlichen Korrelationsmetriken oder einer Kombination davon beinhalten; und
Bestimmen eines oder mehrerer Indizes, die einen Grad der Empfindlichkeit der Probe gegenüber der Konzentration des einen oder der mehreren antimikrobiellen Mittel darstellen, die an jeder Stelle bereitgestellt werden, unter Verwendung der einen oder der mehreren Metriken für die mindestens eine Region jeder Stelle aus einem oder mehreren Indizes;
wobei das topographische Oberflächenprofil einen Höhenwert für jedes Pixel beinhaltet, das innerhalb der assoziierten Region angeordnet ist; und
die eine oder die mehreren Metriken unter Verwendung des Höhenwerts für jedes Pixel bestimmt werden, das innerhalb der assoziierten Region angeordnet ist.

2. Verfahren nach Anspruch 1, wobei der eine oder die mehreren Indizes einen oder mehrere qualitative Indizes beinhalten, wobei der eine oder die mehreren qualitativen Indizes einen ersten Index beinhalten, der angibt, dass die Probe gegenüber der Konzentration des einen oder der mehreren antimikrobiellen Mittel empfindlich ist, und einen zweiten Index, der angibt, dass die Probe gegen die Konzentration des einen oder der mehreren antimikrobiellen Mittel resistent ist, wobei optional der eine oder die mehreren qualitativen Indizes einen dritten Index beinhalten, der angibt, dass die Probe heteroresistent gegen die Konzentration des einen oder der mehreren antimikrobiellen Mittel ist.

3. Verfahren nach einem der Ansprüche 1 oder 2, wobei das eine oder die mehreren antimikrobiellen Mittel ein oder mehrere Antibiotika beinhalten.

4. Verfahren nach einem der Ansprüche 1-3, wobei jede Stelle ein Kulturmedium beinhaltet, wobei das Kulturmedium optional ein festes Medium und/oder ein flüssiges Medium beinhaltet.

5. Verfahren nach Anspruch 4, wobei das Kulturmedium ein Agar-Medium beinhaltet.

6. Verfahren nach einem der Ansprüche 1-5, wobei das Bestimmen der einen oder der mehreren Metriken Pixel der assoziierten Region eines topographischen Bildes verwendet, das von einer optischen Bildgebungsvorrichtung erfasst wird.

7. Verfahren nach einem der Ansprüche 1-6, wobei die eine oder die mehreren volumetrischen Metriken ein Gesamtvolumen mindestens einer Region jeder Stelle beinhalten.

8. Verfahren nach einem der Ansprüche 1-7, wobei die eine oder die mehreren Verteilungsmetriken Kurtosis beinhalten.

9. Verfahren nach einem der Ansprüche 1-8, wobei der eine oder die mehreren Indizes basierend auf einer biophysikalischen Beziehung zwischen der einen oder den mehreren Metriken und dem einen oder den mehreren Indizes bestimmt werden.

10. Verfahren nach einem der Ansprüche 1-9, wobei die eine oder die mehreren Stellen mindestens eine Stelle und/oder mindestens einen Abschnitt beinhalten, die/der die Probe ohne das eine oder die mehreren antimikrobiellen Mittel beinhaltet, wobei das Verfahren ferner Folgendes umfasst:
Bestimmen einer oder mehrerer Metriken der mindestens einen Stelle und/oder des mindestens einen Abschnitts, die/der die Probe ohne das eine oder die mehreren antimikrobiellen Mittel beinhaltet, wobei die eine oder die mehreren Metriken unter anderem eine oder mehrere von volumetrischen Metriken, Verteilungsmetriken, räumlichen Korrelationsmetriken oder einer Kombination davon beinhalten;
wobei das Bestimmen des einen oder der mehreren Indizes Vergleichen der einen oder der mehreren Metriken der einen oder der mehreren Stellen, die die Probe und das eine oder die mehreren antimikrobiellen Mittel beinhalten, mit der einen oder den mehreren Metriken der mindestens einen Stelle und/oder des mindestens einen Abschnitts beinhaltet, die/der die Probe ohne das eine oder die mehreren antimikrobiellen Mittel beinhaltet.

11. Verfahren nach einem der Ansprüche 1-10, wobei jede Stelle (i) einen oder mehrere Testabschnitte beinhaltet, wobei jeder Testabschnitt die Probe und eine Konzentration eines oder mehrerer antimikrobieller Mittel aufweist, die auf einem Kulturmedium angeordnet sind, und/oder (ii) einen oder mehrere Kontrollabschnitte, die das Kulturmedium ohne das eine oder die mehreren antimikrobiellen Mittel aufweisen.

12. Verfahren nach Anspruch 11, wobei der eine oder die mehreren Kontrollabschnitte einen oder mehrere Kontrollabschnitte mit dem Kulturmedium ohne das eine oder die mehreren antimikrobiellen Mittel und die Probe beinhalten.

13. Verfahren nach Anspruch 12, ferner umfassend:
Erhalten topographischer Rohdaten einer oder mehrerer Regionen des Testabschnitts und/oder des Steuerabschnitts unter Verwendung optischer Bildgebung;
Kalibrieren der topographischen Rohdaten für die eine oder die mehreren Regionen des Testabschnitts mit den topographischen Rohbilddaten für die eine oder die mehreren Regionen des Kontrollabschnitts; und
Erzeugen des topographischen Profils für eine oder mehrere Regionen des Testabschnitts jeder Stelle, wobei optional das topographische Profil durch eine topographische Karte dargestellt wird.

14. Verfahren nach einem der Ansprüche 1-13, wobei der eine oder die mehreren Indizes einen oder mehrere quantitative und/oder qualitative Indizes beinhalten, wobei optional der eine oder die mehreren quantitativen Indizes unter Verwendung eines oder mehrerer qualitativer Indizes und eines topographischen Profils bestimmt werden, die mit jeder Stelle assoziiert sind.

15. System zum Bestimmen eines Grades der Empfindlichkeit einer Probe gegenüber einem oder mehreren antimikrobiellen Mitteln basierend auf topographischen Oberflächeneigenschaften, umfassend:
mindestens einen Prozessor; und
einen Speicher, wobei auf dem Speicher computerausführbare Anweisungen gespeichert sind, die bei Ausführung durch den mindestens einen Prozessor den mindestens einen Prozessor veranlassen, die folgenden Schritte auszuführen:
Empfangen eines topographischen Oberflächenprofils für eine oder mehrere Regionen (330, 340, 350) jeder Stelle einer oder mehrerer Stellen eines Behälters, wobei die eine oder die mehreren Stellen eine Probe mit einem oder mehreren Mikroorganismen und einer oder mehreren Konzentrationen eines oder mehrerer antimikrobieller Mittel beinhalten, wobei die eine oder die mehreren Regionen mindestens eine Region mit der Probe und einer Konzentration des einen oder der mehreren antimikrobiellen Mittel beinhalten;
Bestimmen einer oder mehrerer Metriken mindestens einer Region der einen oder mehreren Regionen jeder Stelle unter Verwendung des topographischen Oberflächenprofils für jede Stelle, wobei die eine oder die mehreren Metriken unter anderem eine oder mehrere von volumetrischen Metriken, Verteilungsmetriken, räumlichen Korrelationsmetriken oder einer Kombination davon beinhalten; und
Bestimmen eines oder mehrerer Indizes, die einen Grad der Empfindlichkeit der Probe gegenüber der Konzentration des einen oder der mehreren antimikrobiellen Mittel darstellen, die an jeder Stelle bereitgestellt werden, unter Verwendung der einen oder der mehreren Metriken für die mindestens eine Region jeder Stelle aus einem oder mehreren Indizes;
wobei das topographische Oberflächenprofil einen Höhenwert für jedes Pixel beinhaltet, das innerhalb der assoziierten Region angeordnet ist; und
die eine oder die mehreren Metriken unter Verwendung des Höhenwerts für jedes Pixel bestimmt werden, das innerhalb der assoziierten Region angeordnet ist.

16. System nach Anspruch 15, ferner umfassend den Behälter.

17. System nach Anspruch 15 oder 16, wobei das eine oder die mehreren antimikrobiellen Mittel ein oder mehrere Antibiotika beinhalten.

18. System nach einem der Ansprüche 15-17,
wobei jede Stelle ein Kulturmedium beinhaltet,
wobei optional das Kulturmedium ein festes Medium und/oder ein flüssiges Medium beinhaltet.

19. System nach einem der Ansprüche 15-18, wobei das Bestimmen der einen oder der mehreren Metriken Pixel der assoziierten Region eines topographischen Bildes verwendet, das durch eine optische Bildgebungsvorrichtung erfasst wird.

20. System nach einem der Ansprüche 15-19, wobei die eine oder die mehreren volumetrischen Metriken ein Gesamtvolumen mindestens einer Region jeder Stelle beinhalten.

21. System nach einem der Ansprüche 15-20, wobei die eine oder die mehreren Stellen mindestens eine Stelle und/oder mindestens einen Abschnitt beinhalten, die/der die Probe ohne das eine oder die mehreren antimikrobiellen Mittel beinhaltet, wobei die computerausführbaren Anweisungen ferner den mindestens einen Prozessor veranlassen, die folgenden Schritte auszuführen:
Bestimmen einer oder mehrerer Metriken der mindestens einen Stelle und/oder des mindestens einen Abschnitts, die/der die Probe ohne das eine oder die mehreren antimikrobiellen Mittel beinhaltet, wobei die eine oder die mehreren Metriken unter anderem eine oder mehrere von volumetrischen Metriken, Verteilungsmetriken, räumlichen Korrelationsmetriken oder einer Kombination davon beinhalten;
wobei das Bestimmen des einen oder der mehreren Indizes Vergleichen der einen oder der mehreren Metriken der einen oder der mehreren Stellen, die die Probe und das eine oder die mehreren antimikrobiellen Mittel beinhalten, mit der einen oder den mehreren Metriken der mindestens einen Stelle und/oder des mindestens einen Abschnitts beinhaltet, die/der die Probe ohne das eine oder die mehreren antimikrobiellen Mittel beinhaltet.

22. System nach einem der Ansprüche 15-21,
wobei jede Stelle (i) einen oder mehrere Testabschnitte beinhaltet, wobei jeder Testabschnitt die Probe und eine Konzentration eines oder mehrerer antimikrobieller Mittel aufweist, die auf einem Kulturmedium angeordnet sind, und/oder (ii) einen oder mehrere Kontrollabschnitte, die das Kulturmedium ohne das eine oder die mehreren antimikrobiellen Mittel aufweisen; wobei optional der eine oder die mehreren Kontrollabschnitte einen oder mehrere Kontrollabschnitte mit dem Kulturmedium ohne das eine oder die mehreren antimikrobiellen Mittel und die Probe beinhalten.

23. System nach Anspruch 22, wobei die computerausführbaren Anweisungen ferner den mindestens eine Prozessor veranlassen, die folgenden Schritte auszuführen:
Erhalten topographischer Rohdaten einer oder mehrerer Regionen des Testabschnitts und/oder des Steuerabschnitts unter Verwendung optischer Bildgebung;
Kalibrieren der topographischen Rohdaten für die eine oder die mehreren Regionen des Testabschnitts mit den topographischen Rohbilddaten für die eine oder die mehreren Regionen des Kontrollabschnitts; und
Erzeugen des topographischen Profils für eine oder mehrere Regionen des Testabschnitts jeder Stelle, wobei optional das topographische Profil durch eine topographische Karte dargestellt wird.

24. System nach einem der Ansprüche 15-23,
wobei der eine oder die mehreren Indizes einen oder mehrere von quantitativen und/oder qualitativen Indizes beinhalten,
wobei optional der eine oder die mehreren quantitativen Indizes unter Verwendung eines oder mehrerer qualitativer Indizes und eines topographischen Profils bestimmt werden, die mit jeder Stelle assoziiert sind,
wobei optional der eine oder die mehreren qualitativen Indizes einen ersten Index beinhalten, der angibt, dass die Probe gegenüber der Konzentration des einen oder der mehreren antimikrobiellen Mittel empfindlich ist, und einen zweiten Index, der angibt, dass die Probe gegen die Konzentration des einen oder der mehreren antimikrobiellen Mittel resistent ist, wobei optional der eine oder die mehreren qualitativen Indizes einen dritten Index beinhalten, der angibt, dass die Probe heteroresistent gegen die Konzentration des einen oder der mehreren antimikrobiellen Mittel ist.

## Revendications

1. Procédé de détermination d'un niveau de sensibilité d'un échantillon à un ou plusieurs agents antimicrobiens sur la base de propriétés topographiques de surface, comprenant :
l'obtention d'un récipient (110) comportant un ou plusieurs sites, le ou les sites comportant un échantillon ayant un ou plusieurs microorganismes et une ou plusieurs concentrations d'un ou plusieurs agents antimicrobiens ;
la réception d'un profil topographique de surface pour une ou plusieurs régions (330, 340, 350) de chaque site, la ou les régions comportant au moins une région ayant l'échantillon et une concentration du ou des agents antimicrobiens ;
la détermination d'une ou plusieurs métriques d'au moins une région parmi la ou les régions de chaque site au moyen du profil topographique de surface pour chaque site, la ou les métriques comportant une ou plusieurs métriques parmi des métriques volumétriques, des métriques de distribution, des métriques de corrélation spatiale, entre autres, ou une combinaison de celles-ci ; et
la détermination d'un ou plusieurs indices représentant un niveau de sensibilité de l'échantillon à la concentration du ou des agents antimicrobiens fournis dans chaque site au moyen de la ou des métriques pour l'au moins une région de chaque site à partir d'un ou plusieurs indices ;
dans lequel le profil topographique de surface comporte une valeur de hauteur pour chaque pixel disposé à l'intérieur de la région associée ; et
la ou les métriques sont déterminées au moyen de la valeur de hauteur pour chaque pixel disposé à l'intérieur de la région associée.

2. Procédé de la revendication 1, dans lequel le ou les indices comportent un ou plusieurs indices qualitatifs, le ou les indices qualitatifs comportant un premier indice indiquant que l'échantillon est sensible à la concentration du ou des agents antimicrobiens et un deuxième indice indiquant que l'échantillon est résistant à la concentration du ou des agents antimicrobiens, éventuellement dans lequel le ou les indices qualitatifs comportent un troisième indice indiquant que l'échantillon est hétérorésistant à la concentration du ou des agents antimicrobiens.

3. Procédé de l'une ou l'autre des revendications 1 et 2, dans lequel le ou les agents antimicrobiens comportent un ou plusieurs antibiotiques.

4. Procédé de l'une quelconque des revendications 1 à 3, dans lequel chaque site comporte un milieu de culture, éventuellement dans lequel le milieu de culture comporte un milieu solide et/ou un milieu liquide.

5. Procédé de la revendication 4, dans lequel le milieu de culture comporte un milieu gélosé.

6. Procédé de l'une quelconque des revendications 1 à 5, dans lequel la détermination de la ou des métriques utilise des pixels de la région associée d'une image topographique acquise par un dispositif d'imagerie optique.

7. Procédé de l'une quelconque des revendications 1 à 6, dans lequel la ou les métriques volumétriques comportent un volume total d'au moins une région de chaque site.

8. Procédé de l'une quelconque des revendications 1 à 7, dans lequel la ou les métriques de distribution comportent un aplatissement.

9. Procédé de l'une quelconque des revendications 1 à 8, dans lequel le ou les indices sont déterminés sur la base d'une relation biophysique entre la ou les métriques et le ou les indices.

10. Procédé de l'une quelconque des revendications 1 à 9, dans lequel le ou les sites comportent au moins un site et/ou au moins une section qui comportent l'échantillon sans le ou les agents antimicrobiens, le procédé comprenant en outre :
la détermination d'une ou plusieurs métriques de l'au moins un site et/ou de l'au moins une section qui comportent l'échantillon sans le ou les agents antimicrobiens, la ou les métriques comportant une ou plusieurs métriques parmi des métriques volumétriques, des métriques de distribution, des métriques de corrélation spatiale, entre autres, ou une combinaison de celles-ci ;
dans lequel la détermination du ou des indices comporte la comparaison de la ou des métriques du ou des sites comportant l'échantillon et le ou les agents antimicrobiens à la ou aux métriques de l'au moins un site et/ou de l'au moins une section qui comportent l'échantillon sans le ou les agents antimicrobiens.

11. Procédé de l'une quelconque des revendications 1 à 10, dans lequel chaque site comporte (i) une ou plusieurs sections de test, chaque section de test ayant l'échantillon et une concentration d'un ou plusieurs agents antimicrobiens disposés sur un milieu de culture et/ou (ii) une ou plusieurs sections témoins ayant le milieu de culture sans le ou les agents antimicrobiens.

12. Procédé de la revendication 11, dans lequel la ou les sections témoins comportent une ou plusieurs sections témoins ayant le milieu de culture sans le ou les agents antimicrobiens ni l'échantillon.

13. Procédé de la revendication 12, comprenant en outre :
l'obtention de données topographiques brutes d'une ou plusieurs régions de la section de test et/ou de la section témoin par imagerie optique ;
l'étalonnage des données topographiques brutes pour la ou les régions de la section de test avec les données topographiques brutes d'imagerie pour la ou les régions de la section témoin ; et
la génération du profil topographique pour une ou plusieurs régions de la section de test de chaque site, le profil topographique étant éventuellement représenté par une carte topographique.

14. Procédé de l'une quelconque des revendications 1 à 13, dans lequel le ou les indices comportent un ou plusieurs indices parmi des indices quantitatifs et/ou qualitatifs, éventuellement dans lequel le ou les indices quantitatifs sont déterminés au moyen d'un ou plusieurs indices qualitatifs et d'un profil topographique associés à chaque site.

15. Système destiné à déterminer un niveau de sensibilité d'un échantillon à un ou plusieurs agents antimicrobiens en se basant sur des propriétés topographiques de surface, comprenant :
au moins un processeur ; et
une mémoire, mémoire sur laquelle sont stockées des instructions exécutables par ordinateur qui, lors de leur exécution par l'au moins un processeur, amènent l'au moins un processeur à effectuer les étapes suivantes :
réception d'un profil topographique de surface pour une ou plusieurs régions (330, 340, 350) de chaque site parmi un ou plusieurs sites d'un récipient, le ou les sites comportant un échantillon ayant un ou plusieurs microorganismes et une ou plusieurs concentrations d'un ou plusieurs agents antimicrobiens, la ou les régions comportant au moins une région ayant l'échantillon et une concentration du ou des agents antimicrobiens ;
détermination d'une ou plusieurs métriques d'au moins une région parmi la ou les régions de chaque site au moyen du profil topographique de surface pour chaque site, la ou les métriques comportant une ou plusieurs métriques parmi des métriques volumétriques, des métriques de distribution, des métriques de corrélation spatiale, entre autres, ou une combinaison de celles-ci ; et
détermination d'un ou plusieurs indices représentant un niveau de sensibilité de l'échantillon à la concentration du ou des agents antimicrobiens fournis dans chaque site au moyen de la ou des métriques pour l'au moins une région de chaque site à partir d'un ou plusieurs indices ;
dans lequel le profil topographique de surface comporte une valeur de hauteur pour chaque pixel disposé à l'intérieur de la région associée ; et
la ou les métriques sont déterminées au moyen de la valeur de hauteur pour chaque pixel disposé à l'intérieur de la région associée.

16. Système de la revendication 15, comprenant en outre le récipient.

17. Système de la revendication 15 ou 16, dans lequel le ou les agents antimicrobiens comportent un ou plusieurs antibiotiques.

18. Système de l'une quelconque des revendications 15 à 17,
dans lequel chaque site comporte un milieu de culture,
éventuellement dans lequel le milieu de culture comporte un milieu solide et/ou un milieu liquide.

19. Système de l'une quelconque des revendications 15 à 18, dans lequel la détermination de la ou des métriques utilise des pixels de la région associée d'une image topographique acquise par un dispositif d'imagerie optique.

20. Système de l'une quelconque des revendications 15 à 19, dans lequel la ou les métriques volumétriques comportent un volume total d'au moins une région de chaque site.

21. Système de l'une quelconque des revendications 15 à 20, dans lequel le ou les sites comportent au moins un site et/ou au moins une section qui comportent l'échantillon sans le ou les agents antimicrobiens, les instructions exécutables par ordinateur amenant en outre l'au moins un processeur à effectuer les étapes suivantes :
détermination d'une ou plusieurs métriques de l'au moins un site et/ou de l'au moins une section qui comportent l'échantillon sans le ou les agents antimicrobiens, la ou les métriques comportant une ou plusieurs métriques parmi des métriques volumétriques, des métriques de distribution, des métriques de corrélation spatiale, entre autres, ou une combinaison de celles-ci ;
dans lequel la détermination du ou des indices comporte la comparaison de la ou des métriques du ou des sites comportant l'échantillon et le ou les agents antimicrobiens à la ou aux métriques de l'au moins un site et/ou de l'au moins une section qui comportent l'échantillon sans le ou les agents antimicrobiens.

22. Système de l'une quelconque des revendications 15 à 21,
dans lequel chaque site comporte (i) une ou plusieurs sections de test, chaque section de test ayant l'échantillon et une concentration d'un ou plusieurs agents antimicrobiens disposés sur un milieu de culture et/ou (ii) une ou plusieurs sections témoins ayant le milieu de culture sans le ou les agents antimicrobiens ; éventuellement dans lequel la ou les sections témoins comportent une ou plusieurs sections témoins ayant le milieu de culture sans le ou les agents antimicrobiens ni l'échantillon.

23. Système de la revendication 22, dans lequel les instructions exécutables par ordinateur amènent en outre l'au moins un processeur à effectuer les étapes suivantes :
obtention de données topographiques brutes d'une ou plusieurs régions de la section de test et/ou de la section témoin par imagerie optique ;
étalonnage des données topographiques brutes pour la ou les régions de la section de test avec les données topographiques brutes d'imagerie pour la ou les régions de la section témoin ; et
génération du profil topographique pour une ou plusieurs régions de la section de test de chaque site, le profil topographique étant éventuellement représenté par une carte topographique.

24. Système de l'une quelconque des revendications 15 à 23,
dans lequel le ou les indices comportent un ou plusieurs indices parmi des indices quantitatifs et/ou qualitatifs, éventuellement dans lequel le ou les indices quantitatifs sont déterminés au moyen d'un ou plusieurs indices qualitatifs et d'un profil topographique associés à chaque site,
éventuellement dans lequel le ou les indices qualitatifs comportent un premier indice indiquant que l'échantillon est sensible à la concentration du ou des agents antimicrobiens et un deuxième indice indiquant que l'échantillon est résistant à la concentration du ou des agents antimicrobiens, éventuellement dans lequel le ou les indices qualitatifs comportent un troisième indice indiquant que l'échantillon est hétérorésistant à la concentration du ou des agents antimicrobiens.
